# EUROPEAN PATENT SPECIFICATION

(11) **EP 3 506 843 B1**
(45) Date of publication and mention of the grant of the patent: **07.08.2024**
(21) Application number: 17765111.4
(22) Date of filing: 30.08.2017
(51) Int. Cl.: A61B 17/34

(54) **LAPAROSCOPY TROCAR**
LAPAROSKOPIETROKAR
TROCART DE LAPAROSCOPIE

(30) Priority: 30.08.2016 GB 201614658
(43) Date of publication of application: 10.07.2019
(73) Proprietor: Carewill, Lda., 4460-439 Porto (PT)
(72) Inventor: MONTEIRO, Paulo Alexandre Pinto, 4450-349 Leça da Palmeira-Porto (PT)
(74) Representative: Edson, Russell Gregory
(86) International application number: PCT/EP2017/071767
(87) International publication number: WO 2018/041897

(56) References cited:
- US-A- 5 248 298
- US-A- 5 364 372
- US-A1- 2005 077 689
- US-A1- 2006 253 024
- US-A1- 2010 081 988
- US-A1- 2011 166 527
- US-B1- 7 846 134

## Description

### FIELD OF THE INVENTION

The present invention relates to a laparoscopy trocar for minimally invasive surgical procedures. In particular the invention relates to an improved disposable laparoscopy trocar for use in accessing the abdominal cavity of a patient.

### BACKGROUND TO THE INVENTION

Laparoscopy is a well-known surgical procedure in which one or more small incisions is made in the abdominal cavity, and a pressurised gas, usually carbon dioxide, is used to inflate the abdominal cavity. This act can be commonly referred to as insufflation. Insufflation creates a cavity in which the surgeon can more freely access and view the internal organs. A cannula is inserted through an incision to provide a path into the abdominal cavity. A laparoscope is inserted through the cannula to view the internal cavity and organs during the surgical procedure, which allows the surgeon to view inside the cavity. The cannula generally comprises an elongate tube which traverses the abdominal wall and comprises at least one valve seal, through which instruments can be introduced into the cavity, and which seals around the shaft of the instruments to maintain the pressurised gas within the cavity during the procedure. Second valve seals can be included to improve the sealing function in both the presence of, and in the absence of, surgical tools or instruments within the cannula.

An obturator is used inside the cannula, during insertion of the cannula, in order to drive the cannula through the incision made by the surgeon. The obturator generally has a pointed end in order to drive the sides of the incision apart, to accommodate the cannula. In most cases, the obturator is inserted into the cannula in the same manner as a surgical tool would be inserted, and so is accommodated in the bore of the cannula. The obturator generally has some form of tapered point, which acts to separate the wall as the cannula is introduced. The obturator can be hollow in order to accommodate a laparoscope. Further, at least the tip can be transparent, so that when introducing the trocar through the incision, it is possible for the surgeon to view the layers of the abdominal wall during insertion of the obturator into the abdominal cavity.

The cannula is provided with a source of pressurised fluid, generally a substantially inert gas such as CO₂. The source of gas is controlled via a valve situated on the cannula, which can be opened to allow gas into the abdomen and closed to stop the flow of gas into the abdomen. The fluid valve on the cannula is generally operated manually by the user to release fluid into the abdominal cavity, or prohibit the fluid from entering the abdominal cavity under pressure. Converesely, the valve can be opened to release fluid from the cavity and closed to retain fluid within the cavity when no pressurised fluid source is connected.

US2010/081988A1 describes a system comprising a trocar which is suitable as a first entry surgical access system. US5248298A describes an insert for a shielded trocar that is actuated by a surgical trocar cannula handle. US7846134B1 describes a flexible walled cannula which deforms, allowing the use of larger gauge surgical instruments while providing a self-sealing incision. US2011/166527A1 describes a hemostatic valve apparatus, comprising a valve conduit with first and second fixed ends and a rotatable member positioned between the first and second ends.

### SUMMARY OF THE INVENTION

The present invention provides a laparoscopy trocar as defined by claim 1, comprising:
a cannula comprising:
   first and second ends and a bore extending therebetween;
   at least one flexible seal disposed in the bore, for inhibiting the passage of pressurised gas from the first end to the second end of the cannula; and
   a fluid control valve configured to selectively open and close a fluid communication path for carrying fluid from a point external to the bore, into the bore of the cannula;
      and
an obturator, comprising:
   a tip, a body, and a shaft connecting the tip to the body, wherein the body of the obturator comprises control means configured to cause the fluid control valve to move from its open to its closed position.

The control means may be configured to cause the fluid control valve to close when the obturator is inserted into the cannula.

The control means may comprise mechanical means configured to mechanically actuate the fluid control valve.

The control means may be configured to actuate a manipulable handle of the fluid control valve.

The control means may be configured to prevent actuation of the fluid control valve when the obturator is placed in full engagement with the cannula.

The control means may be disposed in a handle portion of the obturator.

The fluid control valve may be disposed in a handle portion of the cannula.

The respective handle portions of the cannula and the obturator may be configured to align with one another when the obturator is fully inserted in the cannula, the handle of the cannula being at least partially received, optionally fully received, in the handle of the obturator.

The respective handle portions of the cannula and the obturator may be configured to enclose an input portion of the fluid control valve to prevent external actuation of the input portion.

The control means may comprise at least one sloped portion provided in the handle portion of the obturator, the sloped portion being configured to drive the input portion of the fluid control valve laterally relative to the axis of the bore of the cannula, to actuate the fluid control valve.

The sloped portion may be configured to rotate the input portion of the fluid control valve to actuate the fluid control valve.

The control means may comprise a pair of sloped portions configured to engage opposite sides of the input portion to actuate the fluid control valve.

The tip of the obturator may furthermore comprise a pair of concave front surfaces meeting at an acute angle to form an acutely angled front edge in a plane substantially parallel to a longitudinal axis of the shaft.

The tip of the obturator may further comprise a convex rear face substantially opposite the front edge.

The rear face may be oriented so as to be convex in a direction away from the longitudinal axis of the shaft

The plane of the front edge of tip may be oriented in a first direction away from a longitudinal axis of the shaft, the first direction being substantially parallel to a longitudinal axis of the handle portion of the obturator.

The invention provides an obturator as defined by claim 12.

The invention further provides a method of manufacturing a laparoscopy trocar or an obturator as defined by claim 13.

The invention further provides a computer-readable medium as defined by claim 14.

### BRIEF DESCRIPTION OF THE DRAWINGS

Embodiments of the invention will now be described with reference to the accompanying drawings, in which:
Figures 1A to 1H illustrate a cannula of the laparoscopy trocar of the invention;
Figures 2A to 2F illustrate an obturator of a laparoscopy trocar according to one embodiment of the invention;
Figures 3A to 3E illustrate the process of assembling the cannula and obturator together, and the trocar in its assembled state.

### DETAILED DESCRIPTION OF EMBODIMENT(S)

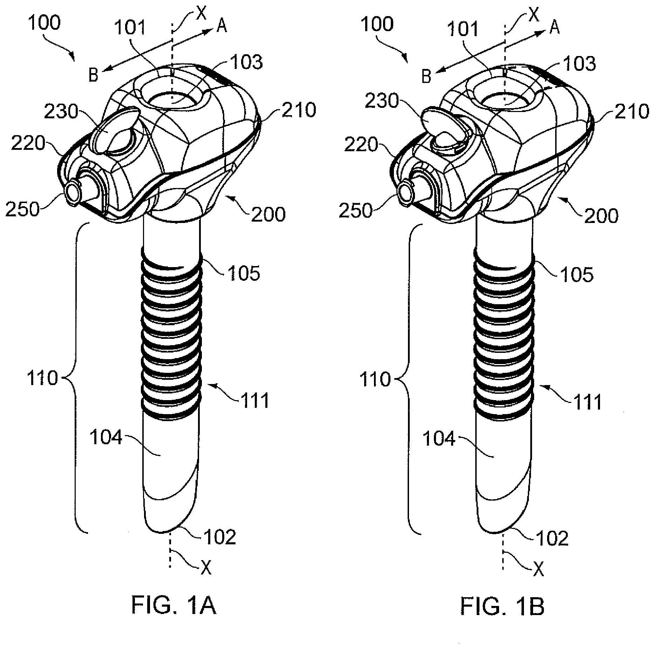

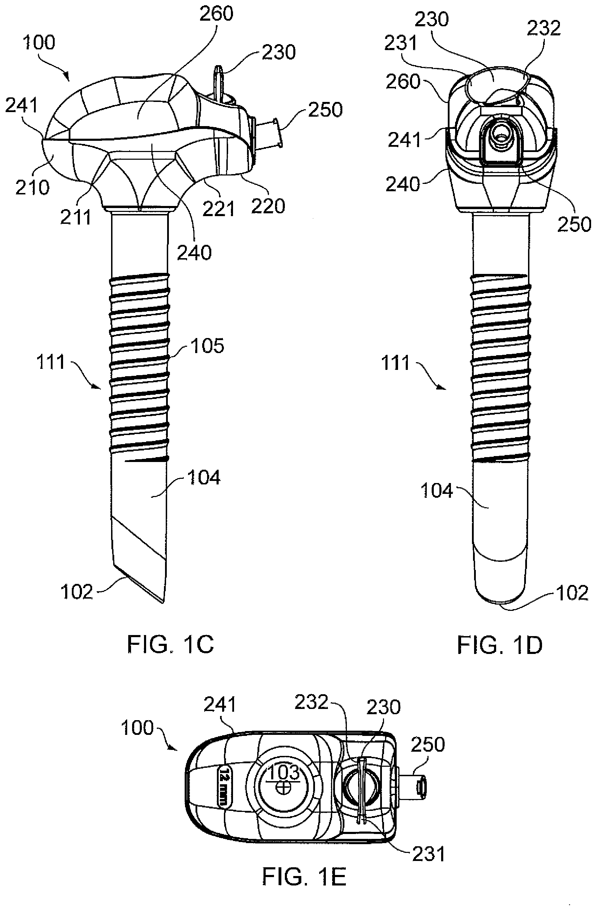

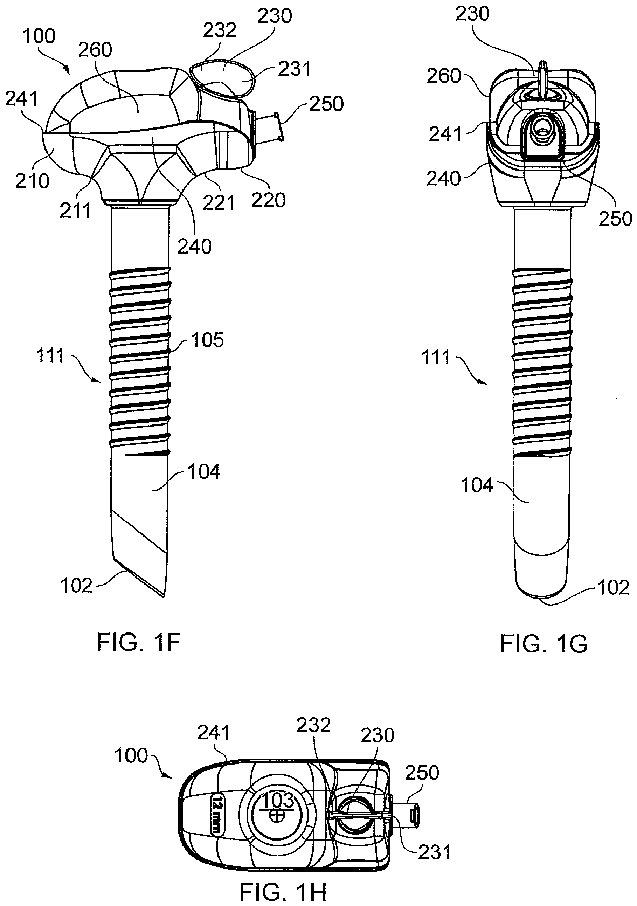

Figures 1A to 1H illustrate a cannula suitable for use in embodiments of a laparoscopy trocar. The cannula has a first open end 101 and a second open end 102. An open bore 103 extends between the first 101 and second 102 ends. The bore 103 is surrounded by a cannula wall 104. The cannula wall 104 can be provided with external ridges 105, which may be of a ring form, or helical as illustrated, but generally extend circumferentially with respect to the wall 104. These ridges assist with securely locating the cannula 100 in the abdominal wall once the cannula has been inserted in an incision in the abdominal wall. The cannula 100 comprises a handle portion 200. The handle portion 200 is located toward the first end 101 of the cannula. The handle portion 200 generally has an outer diameter which is greater than the outer diameter of the cannula wall 104 has in the region of the tubular portion 110 of the cannula 100. The handle portion 200 extends laterally to a longitudinal axis X of the bore 103 in two substantially opposing directions, generally indicated by arrows A and B. The direction of arrow A can generally be considered a rearward direction, while the direction of arrow B can be considered a forward direction, although this terminology is not intended to be limiting and its use is merely illustrative of the two different directions, as will be appreciated upon a full reading of the subsequent description. The handle 200 therefore has a rearward extending handle portion 210 and a forward extending handle portion 220. The cannula 100 also comprises a fluid inlet 250. The fluid inlet 250 is generally configured to permit a fluid, normally a pressurised gas such as CO₂, to enter the bore 103 of the cannula 100 through a wall 104 of the cannula. The fluid inlet 250 can be opened or closed by valve means, which can take any known form. The illustrated embodiment generally takes the preferred form of a rotatable tap, which can be switched on or off to open or close the fluid path from the fluid inlet 250 to the bore 103 of the cannula. This item can therefore be generally termed a fluid control valve. The fluid control valve may be provided with a manipulable handle 230. The handle 230 can be manipulated to move the fluid control valve from an open position to a closed position. As such, a user of the cannula 100 can manually switch a fluid flow path from the fluid inlet 250 to the bore 103 on and off. A source of pressurised CO₂ is commonly attached to a fluid inlet such as fluid inlet 250, to inflate the abdominal cavity of a patient once the cannula 100 has been inserted through an incision in the abdomen of the patient. Figure 1B illustrates the handle 230 of the fluid control valve having been rotated through substantially 90 degrees, to move the fluid control valve to a closed configuration, thus sealing the fluid path between the fluid inlet 250 and the bore 103, to prevent fluid flow through the fluid inlet 250. As will be appreciated, the fluid inlet 250 can of course act as a fluid outlet when gas is being vented from the abdominal cavity and merely acts as an inlet when fluid is being provided to the bore 103 through the inlet 250 from a suitably pressurised fluid source. More generally, inlet 250 may therefore be termed a fluid port.

Figure 1C shows a side view of the cannula 100 with the fluid control valve in the closed position of figure 1B. As can be more clearly seen in figure 1C, the rearward 210, and forward 220, sections of the handle portion 200 can provide concave portions 211 and 221, which can accommodate fingers of a user, so that fingers can grip the handle portion 200 on either side of the tube portion 111 of the cannula 100. The lower or distal portion 240 of the handle portion 200, located adjacent the tube section 111, may be provided with a slightly larger outer dimension than an upper, or proximal portion 260, which is located proximal to the hand of a user holding the device, and located at an opposite side of the distal portion 240 from the tube portion 111.

These two distal and proximal portions 240 and 260 may be separated by a step, ridge or other, optionally graduated, change in outer dimension of the handle portions 200, 210, 220.

As will be appreciated in relation to later figures, this can allow distal portion 260 of the handle portion 200 to be received in a handle portion of an obturator when the two items are combine, as will be described in relation to later figures. Figure 1E illustrates a top view of the cannula 100, i.e. when viewed from a direction of the first open end 101. In this view, as can be seen, the handle 230 of the fluid control valve is oriented with its principal axis, i.e. that extending between its first 231 and second 232 ends, substantially at a right-angle to an axis of the fluid flow path passing from the fluid inlet 250 to the bore 103 of the cannula 100.

Figures 1F to 1H illustrate the same views as shown in figures 1C to 1E, but with the valve handle 230 in an open position, in which the principal axis of the handle 230 is substantially aligned with the direction of a flow path flowing from the fluid inlet 250 to the bore 103 of the cannula 100.

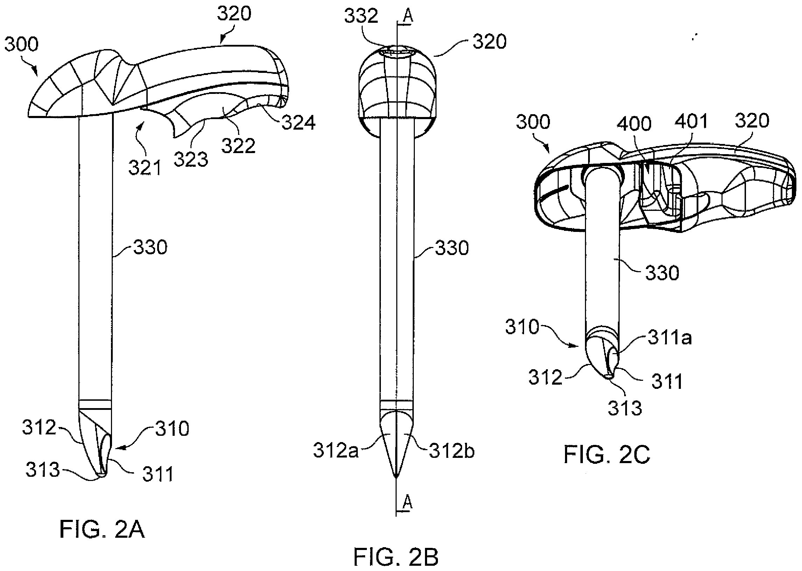

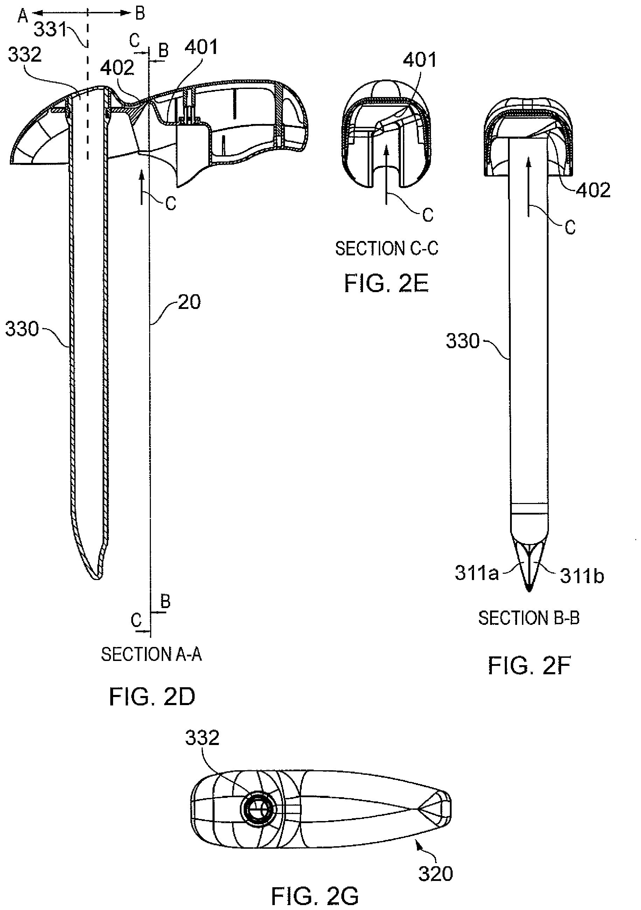

Figures 2A to 2F show an obturator for a laparoscopy trocar in accordance with embodiments of the invention. The obturator 300 generally comprises a tip 310, a body 320 and a shaft 330 extending between the two. The shaft 330 is generally configured to pass within the bore 103 of the cannula 100 of figures 1A to 1H, i.e. by having an outer diameter smaller than the inner diameter of the bore 103. Preferably, the outer diameter of the shaft 330 is a close fit with the inner wall of the bore 103 so as to steady the shaft 330 within the bore 103. The shaft is preferably hollow, having an opening 332 at a proximal end through which a laparoscope can be introduced and inserted into the bore of the shaft 330. Any part of either or both of the shaft 330 and/or the tip 310 of the obturator can be made from a transparent material. This can be a clear thermoplastic material. Therefore, when the trocar is passing through the incision it is possible for the surgeon to visualize the different tissue layers of the abdominal wall via the laparoscope inserted into the bore of the shaft 330. It is preferable for the laparoscope to be inserted into the bore as close as possible to the tip 310 so that the image taken by it is of the best quality and clarity for the surgeon to view.

The body 320 in the illustrated example forms a handle portion of the obturator. As will be described in further detail in relation to later figures, when the obturator 300 is combined with the cannula 100, the handle portion 200 of the cannula 100 and the body 320 of the obturator 300 combine to form an integrated handle portion of the laparoscopy trocar.

The handle portion 320 generally comprises a receiving area 321 for receiving a handle portion 200 of the cannula. An extension portion 322 of the handle portion 320 extends away from the receiving area 321, such that when the handle portion 200 of the cannula and the handle portion 320 of the obturator 300 are combined, an extended handle portion is provided to the overall trocar assembly comprising the obturator 300 and the cannula 100. The extension portion 322 may comprise convex portions 323 and 324 configured to accommodate the fingers of a user when grasping the overall handle of the trocar.

Figures 2C to 2F in particular illustrate one embodiment illustrating a way in which control means for actuating the fluid control valve of the cannula can be implemented. However, a number of different ways in which such control means can be implemented can be envisaged. The general concept devised by the applicant relates to any form of control means being integrated into the obturator so as to cause the fluid control valve of the cannula to switch between its open and closed positions. The preferred principal function of the control means is to cause the fluid control valve to be switched to a closed position when the obturator is inserted into the cannula. This is because it can be advantageous for the fluid control valve to be maintained in a closed position during insertion of the cannula into the abdominal cavity. Therefore, it can be advantageous to cause the combination of the obturator, which is used during the insertion procedure, with the cannula, to cause closure of the fluid control valve. Although the main example illustrated herein comprises a mechanical means for actuation of the fluid control valve by the obturator, it may be envisaged that other means, such as electronic means, be incorporated into the two parts of the device to achieve this aim. For example, sensing means may be provided, in the form of magnets and hall sensors, or RFID transmitters and receivers, or any other form of means for electronically sensing the placement of the obturator in the cannula. Those sensors may transmit a signal to a controller, which then actuates the fluid control valve in response to input from those sensors detecting the presence of the obturator in the cannula.

However, it can be advantageous for the laparoscopy trocar to be manufactured as a disposable single use item. In such cases, it can be preferable to manufacture all components from materials which can be manufactured at low costs, for example by 3D printing, or by injection moulding at high volume, to create a low cost disposable assembly. In these instances, it may be preferable to omit complex electronic components and so a mechanical means for controlling the fluid control valve when the obturator is introduced into the cannula can be preferred, as is illustrated in the embodiments shown in the figures.

In Figure 2C, mechanical control means for the fluid control valve are illustrated in an example where they are provided internally to the handle portion 320 of the obturator 300. A first sloped surface 401 is provided and this can be configured to engage at least a first end 231 of the handle 230 of the fluid control valve. When the handle 230 is partially engaged by the control means 400, i.e. not completely closed neither completely opened, the slope 401 will engage the handle 230 in between the centre or axis of rotation of the handle 230, and the first end 231. Preferably, at the same time, the slope 402 will engage the handle 230 in between its centre or axis of rotation and the second end 232.

The internal workings of the fluid control valve are conventional and so are not illustrated in detail herein in the interests of efficiency of the disclosure. A skilled reader will be familiar with the types of fluid control valve required to switch on and off a CO₂ feed to the internal bore 103 of the cannula 100.

In figure 2D, the sloped surface 401 is cut through in cross section at the location illustrated by section line A-A in Figure 2B. As can be seen in the figure, sloped surface 401 is located substantially to a forward side, in the direction of arrow B, of an axis of rotation of the fluid control valve. A second sloped surface 402 can be provided in addition to, or alternatively to, the sloped surface 401. However, best operation may be achieved by having both of sloped surfaces 40 and 402 in combination.

The second sloped surface 402 is provided to a rearward side of the axis of rotation 20 of the handle 230. As can be seen in figure 2D second sloped surface 402 is located substantially between the axis of rotation of the fluid control valve, which coincides substantially with the section line 20 shown in figure 2D and the axis 331 of the shaft 330. A sloped surface 402, for engaging the fluid control valve can therefore be located substantially between the axis of the shaft 330 and the axis of rotation 20 of the fluid control valve. In the preferred arrangement illustrated, the sloped surfaces 401 and 402, as seen on figure 2D, are located substantially on opposite sides of the axis of the rotation 20 of the handle 230.

Sloped surface 401 may be provided additionally, or alternatively, to sloped surface 402. Sloped surface 401 can be positioned on an opposite side of the axis of rotation 20 of the fluid control valve to the shaft 330. In such an arrangement, the axis 20 of the fluid control valve falls between the axis 331 of the shaft 330 and the sloped surface 401 when the trocar is assembled.

As will be appreciated, therefore, when the handle 230 of the fluid control valve of the cannula 100 is brought into engagement with one and/or the other of the first 401 and second 402 sloped surfaces, the handle 230 will be caused to rotate around its axis 20. In the preferred arrangement illustrated, the control means 400 will engage simultaneously both ends 231 and 232 of the handle 230, on the sloped surfaces 401 and 402 respectively.

This can cause the fluid control valve to be switched between its open and closed configurations. This switching can occur when the cannula is introduced in a direction of arrow C.

Details of the tip 310 of the obturator 300 shown in figures 2A to 2G will be discussed in further detail later.

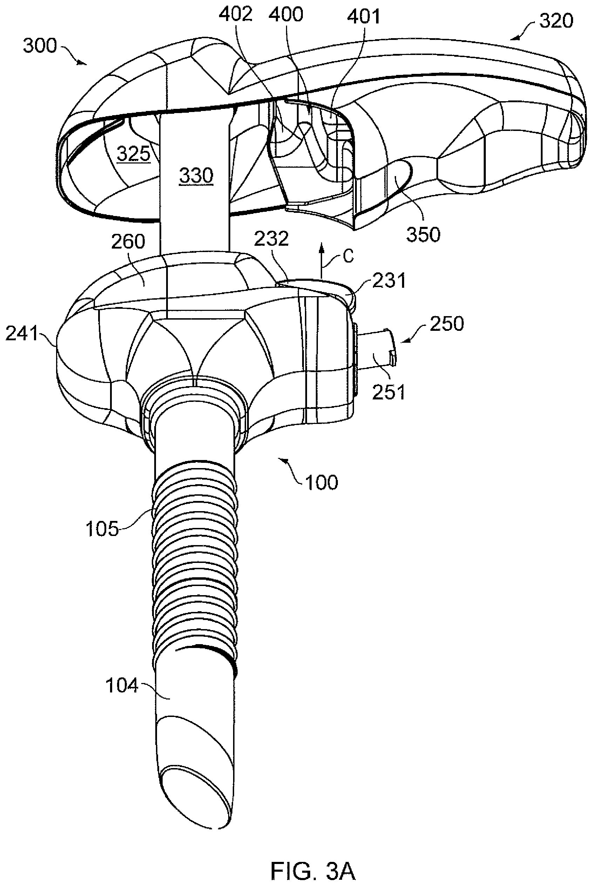

Figure 3A illustrates a perspective view of the obturator 300 being introduced into the cannula 100. As can be seen in figure 3A, the proximal portion 260 of the handle portion 200 of the cannula 100, which has a reduced lateral dimension relative to the distal portion 240 can be received in the cavity 325 of the handle portion 320 of body of the obturator 300.

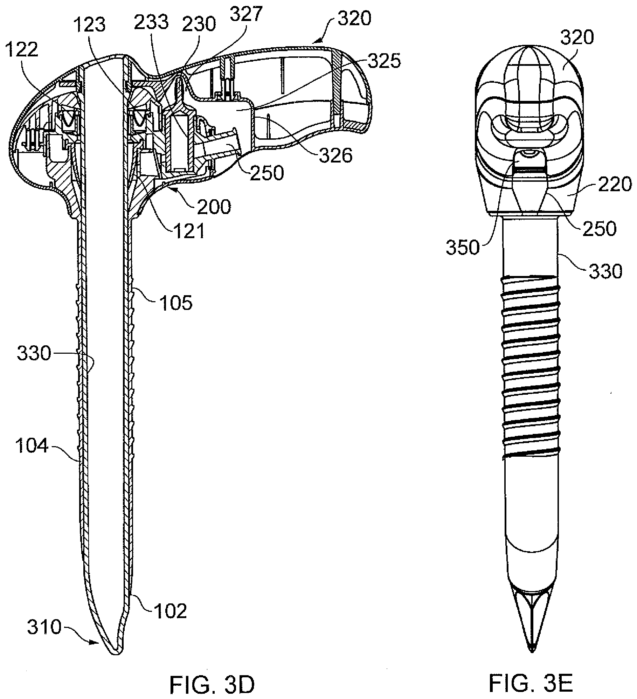

The handle portion 320 can also be configured to include an opening 350 to allow the connector portion 251 of the fluid port 250 to pass at least partially within the handle portion 320 of the obturator. As shown in the preferred embodiment illustrated, the fluid port 250 may be completely housed in the cavity 325 of the handle portion 320, as can be seen in figure 3D. This will prevent the connector port 250 from interfering with the fingers of the user when holding the assembled trocar and thus prevents the port 250 disrupting the ergonomics of the handle of the trocar. This can also help to ensure that the fluid source connected to the connector portion 251 is not inadvertently connected to the cannula prior to the insertion of the cannula into the abdominal cavity. The enclosing of the fluid inlet 250 by the handle portion 320 can also be seen in figure 3E.

As can be seen, when the handle 230 of the fluid control valve is introduced towards the mechanical control means 400, the first 401 and/or second 402 sloped surfaces engage one or the other (preferably both) of the first 231 and/or second 232 ends of the handle 230 of the fluid control valve to actuate the fluid control valve as described above.

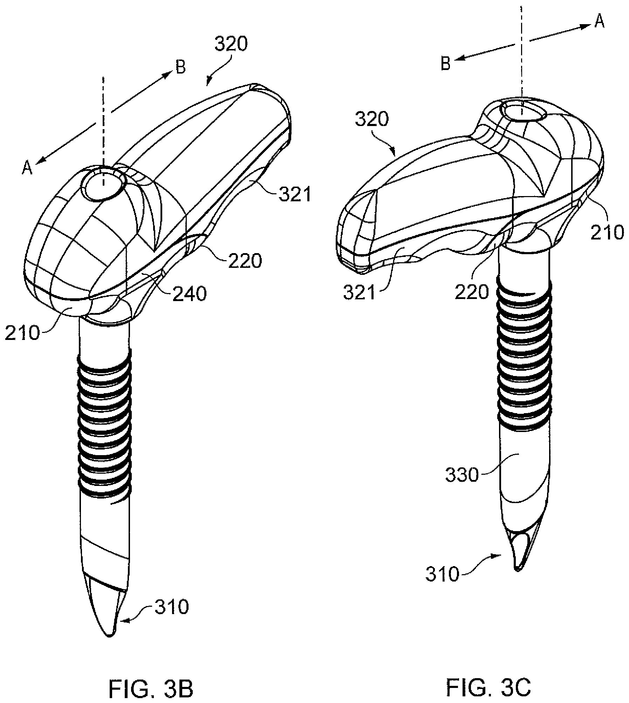

Figures 3B and 3C show perspective views of the laparoscopy trocar with the obturator 300 fully inserted into the cannula 100. As can be seen, the extension portion 321 provides an extended handle portion which extends beyond the extent of the handle portion 200 of the cannula in a direction of arrow B, i.e. a forward direction.

Figure 3D shows a cross section through the assembled laparoscopy trocar. This figure illustrates how the shaft 330 of the obturator is received within the cannula wall 104, with the tip 310 protruding from the second open end 102 of the cannula. The open end is distal from the handle portion 200. Fluid inlet 250 of the cannula can be seen, received in the handle portion 320 of the obturator. A wall 326 of the cavity 325 of the handle portion 320 is located in relatively close proximity to the open end of the fluid inlet 250, which helps prevent damage to the fluid inlet 250 when the obturator 300 is located in the cannula 100 and improves general ergonomic function of the handle by protecting the user's fingers from the inlet 250.

Partial internal detail of the fluid control valve 233 can be seen. However, the full internal workings of the valve are not explained in detail, since these are conventional for a rotational fluid control valve as used in known cannulas. As can be seen in the figure, the handle 230 of the fluid control valve 233 is received in a cavity 327, which encloses the handle portion 230, such that it cannot rotate to its open position. Although the embodiment shown in figure 3D includes a rotational fluid control valve, embodiments can be envisaged in which other forms of mechanical fluid control valve are moved by, and their movement restricted by, the handle portion 320 of the obturator 300. For example, a linear action fluid control valve could be implemented, which could be driven linearly between open and closed positions by introduction of the obturator 300 into the cannula 100. This linear movement can be restricted by suitably configured features of the body or handle portion 320 of the obturator 300. For example, if the cannula 100 is introduced in a linear direction of arrow C, then the linear valve may be directly driven in an opposite direction of arrow C by features of the handle 320 impacting the control valve. Alternatively, sloped surfaces may be used to drive a linear valve substantially laterally relative to the direction C of introduction of the cannula to move it between open and closed positions.

As is conventional, the cannula 100 further comprises one or more valve seals located in the bore 103. The illustrated example comprises two valve seals. A first valve seal 121 is conventionally configured to seal the bore 103 in the absence of any obturator or surgical instrument being present in the bore 103. The second seal 122 is also conventional and is configured to seal around the obturator 300, or any other instrument inserted into the bore 103, while bellows 123, located around the central part of the seal 122, permit some lateral movement of the instrument within the bore 103 whilst allowing a sealing edge of the valve seal 122 to maintain a fluid-tight seal around the outer surface of the inserted instrument. Such first 121 and second 122 seals are conventional in the art and so detail of them is not described herein and any such suitable seal or seals can be used in the cannula 100 of embodiments of the present disclosure.

A further aspect of the laparoscopy trocar described herein, is the form of the tip 310 of the obturator 300. The form of the tip can be seen throughout figures 2A to 2F and 3B to 3E. The tip 310 comprises a front edge 311 and a rear edge or face 312. The tip may further comprise an end 313 which may be substantially rounded or dome-shaped. The front edge 311 is formed where a pair of concave faces 311A and 311B meet at an acute angle. The concavity of the faces causes the front edge 311 to also have a concave form when viewed from a point substantially perpendicular to its plane of curvature, as seen in figure 2A. The front edge 311 is therefore concave with respect to the longitudinal axis 331 of the obturator 300, bowing inwardly toward the axis 331. The front edge may be sharp so as to form a blade, which may be configured to cut through layers of the abdominal wall upon insertion into an initial incision made by a surgeon during insertion of the obturator tip 310.

The rear edge or face 312 of the tip 310 of the obturator is configured so as not to have any sharp edge which would cut the abdominal wall. The rear face is preferably convex, as shown in figures 2A and 2C in particular, i.e. convex with respect to the longitudinal axis 331 of the obturator, or bowing outwardly away from that axis. The rear edge or face 312 may still comprise first and second faces 312A and 312B, or may be formed of a single substantially smooth convex surface. However, if comprising two or more faces, it is preferred that such faces meet at a greater internal angle than the front faces 311A and 311B, so that no bladed edge is created at the rear edge or face 312. If any edge is created, then this is generally formed at least at a greater internal angle than the acute internal angle of the front edge 311 formed by the first and second concave faces 311A and 311B.

A tip configured in such a manner can reduce the amount of friction, and therefore the amount of force, required to insert the obturator into the abdominal cavity. The bladed front face 311 is configured in such a manner that it will can at least partially cut through the layers of the abdominal wall upon insertion. Therefore, a little more trauma may be caused by the tip 310 than if no bladed edge 311 were provided. However, in certain situations blunt edges can cause greater friction and tearing of the abdominal wall depending upon the hardness, or lack thereof, of the abdominal wall. Therefore providing one sharp edge to the obturator can reduce the overall level of trauma caused in certain situations. However, the form of the tip allows a lesser degree of force to be used, which reduces the risk of the trocar penetrating the cavity wall in a less controlled manner and thus decreasing the possibility of injury of internal organs, vessels or other internal abdominal structures by the trocar tip on insertion of the obturator 300 and cannula 100.

As can be appreciated from the figures, a plane of the tip which is substantially aligned with the front edge 311 and the rear edge 312 is substantially parallel with both a longitudinal axis of the handle portion 320, and also with a longitudinal axis 331 of the obturator itself. This fore-aft alignment of the tip 310 with the longitudinal dimension of the handle portion 320 means that the tip 310 and its front and rear edges 311 and 312 are better aligned with the hand of a user when gripping the body or handle portion 320 of the obturator, specifically when combined with the cannula 100. This can improve the overall ergonomics of the laparoscopy trocar, facilitating insertion of the trocar into the abdominal cavity. It can be preferable to have the sharp or front edge 311 oriented in the direction described for the following reasons:
1. When a user such as a surgeon is introducing the trocar into the abdominal cavity, the front of the trocar is always facing the surgeon.
2. The surgeon will normally try to make the longitudinal axis of the trocar perpendicular to the body of the patient, as far as is practicable. In this way the trocar will pass through the least amount of abdominal wall and this reduces the amount of trauma caused to tissue. In practice, the users tend to have the longitudinal axis of the trocar slightly inclined towards themselves.
3. In either case, the trocar introduced perpendicular to the body or with a small degree of inclination towards the user, the load generated by the surgeon on the trocar will be (slightly) higher on the front of the trocar tip than on the rear of the trocar tip.
4. Thus, with the sharp edge, that has a cutting effect, on the front face of the tip, the insertion force needed to go through the abdominal wall is less than if this edge was on the rear face of the tip.
5. Also, as the sharp edge is concave, the cutting of the wall will be reduced to a minimum.

Regarding the rear face 312, the convex rear face is significantly elongated in comparison to the front edge, as this will reduce its angle of insertion and thus concur to minimize the insertion force.

Any part of the obturator, cannula or the laparoscopy trocar assembly described herein may be manufactured by automated manufacturing means and methods. Such means and methods can include material removal techniques, and/or additive manufacturing systems and techniques, which are commonly known as 3D-printing systems and techniques. Such manufacturing methods generally require the creation of a computer readable model of the product to be manufactured. From that virtual three-dimensional model, a computer can derive a set of instructions to instruct a 3D printer to manufacture the product, or indeed for a material removal device to "machine" the product from a block of material, by methods generally known as material removal techniques. Different materials having different properties can be better suited to either additive manufacture, or material removal techniques, but both generally start from a 3D model and generate instructions from the model to control a 3D printer or material removal device (often called a CNC-computer numerically controlled-machining device). Such devices are widely available and are not described herein in the interests of efficiency of the disclosure, but such devices will be well known to the person skilled in the art of such automated manufacturing techniques and apparatus'. Suitable 3D models for generating manufacturing instructions can be general 3D CAD (computer aided design) files and those themselves can be considered a computer programme product suitable for generating instructions for the manufacture of the product by automated manufacturing means. Such models can be interpreted by 3D printing software, or a 3D printer device, in order to manufacture the products. Further, metal removal, or CNC machining software can also interpret the CAD models to create instructions for a material removal device to create the product from a block of solid material. Further, such 3D CAD files can be used to create a form for a mould for manufacturing the device by moulding processes, such as injection moulding. Injection moulding can be the most efficient way of manufacturing the components described herein and so use of the described CAD files representing the product to create moulds for injection moulding of the product is included within the scope of this disclosure.

Although the invention has been described above with reference to one or more preferred embodiments, it will be appreciated that various changes or modifications may be made without departing from the scope of the invention as defined in the appended claims.

## Claims

1. A laparoscopy trocar, comprising:
a cannula (100) comprising:
first and second ends (101, 102) and a bore (103) extending therebetween;
at least one flexible seal (121) disposed in the bore (103), for inhibiting the passage of pressurised gas from the first end (101) to the second end (102) of the cannula (100); and
a fluid control valve (233) configured to selectively open and close a fluid communication path for carrying fluid from a point external to the bore (103), into the bore (103) of the cannula;
and
an obturator (300), comprising:
a tip (310), a body (320), and a shaft (330) connecting the tip (310) to the body (320),
wherein the body (320) of the obturator (300) comprises control means (400) configured to cause the fluid control valve (233) to move from its open to its closed position.

2. A laparoscopy trocar according to claim 1, wherein the control means (400) are configured to cause the fluid control valve (233) to close when the obturator (300) is inserted into the cannula (100).

3. A laparoscopy trocar according to claim 1 or claim 2, wherein the control means (400) comprises mechanical means configured to mechanically actuate the fluid control valve (233).

4. A laparoscopy trocar according to any of the preceding claims, wherein the control means (400) are configured to actuate a manipulable handle (230) of the fluid control valve (233), and wherein the control means (400) are configured to prevent actuation of the fluid control valve (233) when the obturator (300) is placed in the cannula (100).

5. A laparoscopy trocar according to any of the preceding claims, wherein the control means (400) is disposed in a handle portion (320) of the obturator (300), and wherein the fluid control valve (233) is disposed in a handle portion (200) of the cannula (100).

6. A laparoscopy trocar according to claim 5, wherein the respective handle portions (200, 320) of the cannula (100) and the obturator (300) are configured to align with one another when the obturator (300) is fully inserted in the cannula (100), the handle of the cannula (100) being at least partially received in the handle of the obturator (300).

7. A laparoscopy trocar according to any of the preceding claims, wherein the respective handle portions (200, 320) of the cannula (100) and the obturator (300) are configured to enclose an input portion of the fluid control valve (233) to prevent actuation of the input portion.

8. A laparoscopy trocar according to any of claims 5 to 7, wherein the control means (400) comprises at least one sloped portion (401, 402) provided in the handle portion (320) of the obturator (300), the sloped portion (401, 402) being configured to drive the input portion of the fluid control valve (233) laterally relative to the axis of the bore (103) of the cannula (100), to actuate the fluid control valve (233), and wherein the sloped portion (401, 402) is configured to rotate the input portion of the fluid control valve (233) to actuate the fluid control valve (233).

9. A laparoscopy trocar according to claim 8, comprising a pair of sloped portions (401, 402) configured to engage opposite sides of the input portion to actuate the fluid control valve (233).

10. A laparoscopy trocar according to claim 1, wherein the tip (310) of the obturator (300) comprises a pair of concave front surfaces (311A, 311B) meeting at an acute angle to form an acutely angled front edge (311) in a plane substantially parallel to a longitudinal axis of the shaft (330).

11. A laparoscopy trocar according to claim 10, wherein the tip (310) of the obturator (300) further comprises a convex rear face (312) substantially opposite the front edge (311), wherein the rear face (312) is oriented so as to be convex in a direction away from the longitudinal axis of the shaft (330), and wherein the plane of the front edge (311) of tip (310) is oriented in a first direction away from a longitudinal axis of the shaft (330), the first direction being substantially parallel to a longitudinal axis of the handle portion (320) of the obturator (300).

12. An obturator (300) for use with the cannula (100) of any of claims 1 to 11, the obturator (300) comprising a handle portion, a tip (310), a body (320), and a shaft (330) connecting the tip (310) to the body (320), the body (320) of the obturator (300) comprising a control means (400) which comprises at least one sloped portion (401, 402) provided in the handle portion (320) of the obturator (300), the sloped portion (401, 402) being configured to drive the input portion of the fluid control valve (233) of the cannula laterally relative to the axis of the bore (103) of the cannula, to actuate the fluid control valve (233).

13. A method of manufacturing a laparoscopy trocar according to any of claims 1 to 11 or an obturator according to claim 12, comprising the steps of:
providing a computer-readable storage medium having data thereon representing a three-dimensional model suitable for use in manufacturing the laparoscopy trocar or obturator, respectively; and
manufacturing the laparoscopy trocar or obturator, respectively, using instructions contained in the three-dimensional model.

14. A computer-readable medium having data thereon representing a three-dimensional model of the laparoscopy trocar according to any one of claims 1 -11 for the method of manufacturing a laparoscopy trocar according to claim 13, or of the obturator (300) according to claim 12 for the method of manufacturing an obturator according to claim 13.

## Patentansprüche

1. Laparoskopietrokar, umfassend:
eine Kanüle (100), umfassend:
erste und zweite Enden (101, 102) und eine sich dazwischen erstreckende Bohrung (103);
mindestens eine flexible Dichtung (121), die in der Bohrung (103) angeordnet ist, zur Unterbindung des Durchleitens von Druckgas vom ersten Ende (101) zum zweiten Ende (102) der Kanüle (100); und
ein Fluidregelventil (233), das zum selektiven Öffnen und Schließen eines Fluidverbindungswegs zum Transportieren von Fluid von einem außerhalb der Bohrung (103) liegenden Punkt in die Bohrung (103) der Kanüle ausgelegt ist;
und
einen Obturator (300), umfassend:
eine Spitze (310), einen Körper (320) und einen Schaft (330), der die Spitze (310) mit dem Körper (320) verbindet,
wobei der Körper (320) des Obturators (300) Steuermittel (400) umfasst, die dazu ausgelegt sind, eine Bewegung des Fluidregelventils (233) von seiner geöffneten in seine geschlossene Stellung zu bewirken.

2. Laparoskopietrokar nach Anspruch 1, wobei die Steuermittel (400) dazu ausgelegt sind, Schließen des Fluidregelventils (233) zu bewirken, wenn der Obturator (300) in die Kanüle (100) eingeführt wird.

3. Laparoskopietrokar nach Anspruch 1 oder Anspruch 2, wobei das Steuermittel (400) mechanische Mittel umfasst, die zum mechanischen Betätigen des Fluidregelventils (233) ausgelegt sind.

4. Laparoskopietrokar nach einem der vorhergehenden Ansprüche, wobei die Steuermittel (400) zum Betätigen eines manipulierbaren Griffs (230) des Fluidregelventils (233) ausgelegt sind, und wobei die Steuermittel (400) zum Verhindern einer Betätigung des Fluidregelventils (233) ausgelegt sind, wenn der Obturator (300) in der Kanüle (100) platziert wird.

5. Laparoskopietrokar nach einem der vorhergehenden Ansprüche, wobei das Steuermittel (400) in einem Griffabschnitt (320) des Obturators (300) angeordnet ist, und wobei das Fluidregelventil (233) in einem Griffabschnitt (200) der Kanüle (100) angeordnet ist.

6. Laparoskopietrokar nach Anspruch 5, wobei die jeweiligen Griffabschnitte (200, 320) der Kanüle (100) und des Obturators (300) dazu ausgelegt sind, sich zueinander auszurichten, wenn der Obturator (300) vollständig in die Kanüle (100) eingeführt ist, wobei der Griff der Kanüle (100) mindestens teilweise in dem Griff des Obturators (300) aufgenommen ist.

7. Laparoskopietrokar nach einem der vorhergehenden Ansprüche, wobei die jeweiligen Griffabschnitte (200, 320) der Kanüle (100) und des Obturators (300) dazu ausgelegt sind, einen Eingabeabschnitt des Fluidregelventils (233) zu verschließen, um eine Betätigung des Eingabeabschnitts zu verhindern.

8. Laparoskopietrokar nach einem der Ansprüche 5 bis 7, wobei das Steuermittel (400) mindestens einen geneigten Abschnitt (401, 402) umfasst, der im Griffabschnitt (320) des Obturators (300) bereitgestellt ist, wobei der geneigte Abschnitt (401, 402) dazu ausgelegt ist, den Eingabeabschnitt des Fluidregelventils (233) seitlich bezüglich der Achse der Bohrung (103) der Kanüle (100) zu treiben, um das Fluidregelventil (233) zu betätigen, und wobei der geneigte Abschnitt (401, 402) dazu ausgelegt ist, den Eingabeabschnitt des Fluidregelventils (233) zu drehen, um das Fluidregelventil (233) zu betätigen.

9. Laparoskopietrokar nach Anspruch 8, umfassend ein Paar geneigte Abschnitte (401, 402), die dazu ausgelegt sind, gegenüberliegende Seiten des Eingabeabschnitts in Eingriff zu nehmen, um das Fluidregelventil (233) zu betätigen.

10. Laparoskopietrokar nach Anspruch 1, wobei die Spitze (310) des Obturators (300) ein Paar konkave Vorderseiten (311A, 311B) umfasst, die in einem spitzen Winkel aufeinandertreffen, um eine spitzwinklige vordere Kante (311) in einer zu einer Längsachse des Schafts (330) im Wesentlichen parallelen Ebene zu bilden.

11. Laparoskopietrokar nach Anspruch 10, wobei die Spitze (310) des Obturators (300) ferner eine konvexe Rückseite (312) umfasst, die der vorderen Kante (311) im Wesentlichen gegenüberliegt, wobei die Rückseite (312) so orientiert ist, dass sie in einer von der Längsachse des Schafts (330) weg führenden Richtung konvex ist, und wobei die Ebene der vorderen Kante (311) der Spitze (310) in einer von einer Längsachse des Schafts (330) weg führenden ersten Richtung orientiert ist, wobei die erste Richtung im Wesentlichen parallel zu einer Längsachse des Griffabschnitts (320) des Obturators (300) ist.

12. Obturator (300) zur Verwendung mit der Kanüle (100) nach einem der Ansprüche 1 bis 11, wobei der Obturator (300) einen Griffabschnitt, eine Spitze (310), einen Körper (320) und einen Schaft (330) umfasst, der die Spitze (310) mit dem Körper (320) verbindet, wobei der Körper (320) des Obturators (300) ein Steuermittel (400) umfasst, das mindestens einen geneigten Abschnitt (401, 402) umfasst, der im Griffabschnitt (320) des Obturators (300) bereitgestellt ist, wobei der geneigte Abschnitt (401, 402) dazu ausgelegt ist, den Eingabeabschnitt des Fluidregelventils (233) der Kanüle seitlich bezüglich der Achse der Bohrung (103) der Kanüle (100) zu treiben, um das Fluidregelventil (233) zu betätigen.

13. Verfahren zur Herstellung eines Laparoskopietrokars nach einem der Ansprüche 1 bis 11 oder eines Obturators nach Anspruch 12, die folgenden Schritte umfassend:
Bereitstellen eines computerlesbaren Speichermediums, das Daten enthält, die ein dreidimensionales Modell repräsentieren, das zur Verwendung bei der Herstellung des Laparoskopietrokars bzw. Obturators geeignet ist; und
Herstellen des Laparoskopietrokars bzw. Obturators unter Verwendung von Anweisungen, die in dem dreidimensionalen Modell enthalten sind.

14. Computerlesbares Medium, das Daten enthält, die ein dreidimensionales Modell des Laparoskopietrokars nach einem der Ansprüche 1-11 für das Verfahren zur Herstellung eines Laparoskopietrokars nach Anspruch 13, oder des Obturators (300) nach Anspruch 12 für das Verfahren zur Herstellung eines Obturators nach Anspruch 13 repräsentieren.

## Revendications

1. Trocart de laparoscopie, comprenant :
une canule (100) comprenant :
des première et seconde extrémités (101, 102) et un alésage (103) s'étendant entre celles-ci ;
au moins un joint souple (121) disposé dans l'alésage (103), pour empêcher le passage d'un gaz sous pression de la première extrémité (101) à la seconde extrémité (102) de la canule (100) ; et
une soupape de régulation de fluide (233) configurée pour ouvrir et fermer sélectivement une voie de communication de fluide pour transporter un fluide d'un point externe à l'alésage (103), dans l'alésage (103) de la canule ;
et
un obturateur (300), comprenant :
une pointe (310), un corps (320), et un arbre (330) reliant la pointe (310) au corps (320),
dans lequel le corps (320) de l'obturateur (300) comprend un moyen de commande (400) configuré pour amener la soupape de régulation de fluide (233) à passer de sa position ouverte à sa position fermée.

2. Trocart de laparoscopie selon la revendication 1, dans lequel le moyen de commande (400) est configuré pour amener la soupape de régulation de fluide (233) à se fermer lorsque l'obturateur (300) est inséré dans la canule (100).

3. Trocart de laparoscopie selon la revendication 1 ou la revendication 2, dans lequel le moyen de commande (400) comprend un moyen mécanique configuré pour actionner mécaniquement la soupape de régulation de fluide (233).

4. Trocart de laparoscopie selon l'une quelconque des revendications précédentes, dans lequel le moyen de commande (400) est configuré pour actionner une poignée manipulable (230) de la soupape de régulation de fluide (233), et dans lequel le moyen de commande (400) est configuré pour empêcher l'actionnement de la soupape de régulation de fluide (233) lorsque l'obturateur (300) est placé dans la canule (100).

5. Trocart de laparoscopie selon l'une quelconque des revendications précédentes, dans lequel le moyen de commande (400) est disposé dans une partie poignée (320) de l'obturateur (300), et dans lequel la soupape de régulation de fluide (233) est disposée dans une partie poignée (200) de la canule (100).

6. Trocart de laparoscopie selon la revendication 5, dans lequel les parties poignée (200, 320) respectives de la canule (100) et de l'obturateur (300) sont configurées pour s'aligner l'une sur l'autre lorsque l'obturateur (300) est complètement inséré dans la canule (100), la poignée de la canule (100) étant au moins partiellement reçue dans la poignée de l'obturateur (300).

7. Trocart de laparoscopie selon l'une quelconque des revendications précédentes, dans lequel les parties poignée (200, 320) respectives de la canule (100) et l'obturateur (300) sont configurées pour enfermer une partie d'entrée de la soupape de régulation de fluide (233) afin d'empêcher l'actionnement de la partie d'entrée.

8. Trocart de laparoscopie selon l'une quelconque des revendications 5 à 7, dans lequel le moyen de commande (400) comprend au moins une partie inclinée (401, 402) prévue dans la partie poignée (320) de l'obturateur (300), la partie inclinée (401, 402) étant configurée pour entraîner la partie d'entrée de la soupape de régulation de fluide (233) latéralement par rapport à l'axe de l'alésage (103) de la canule (100), afin d'actionner la soupape de régulation de fluide (233), et dans lequel la partie inclinée (401, 402) est configurée pour faire tourner la partie d'entrée de la soupape de régulation de fluide (233) afin d'actionner la soupape de régulation de fluide (233).

9. Trocart de laparoscopie selon la revendication 8, comprenant une paire de parties inclinées (401, 402) configurées pour entrer en prise avec des côtés opposés de la partie d'entrée afin d'actionner la soupape de régulation de fluide (233).

10. Trocart de laparoscopie selon la revendication 1, dans lequel l'extrémité (310) de l'obturateur (300) comprend une paire de surfaces avant concaves (311A, 311B) se rencontrant selon un angle aigu pour former un bord avant à angle aigu (311) dans un plan sensiblement parallèle à un axe longitudinal de l'arbre (330).

11. Trocart de laparoscopie selon la revendication 10, dans lequel l'extrémité (310) de l'obturateur (300) comprend en outre une face arrière convexe (312) sensiblement opposée au bord avant (311), dans lequel la face arrière (312) est orientée de manière à être convexe dans une direction opposée à l'axe longitudinal de l'arbre (330), et dans lequel le plan du bord avant (311) de la pointe (310) est orienté dans une première direction opposée à un axe longitudinal de l'arbre (330), la première direction étant sensiblement parallèle à un axe longitudinal de la partie poignée (320) de l'obturateur (300).

12. Obturateur (300) destiné à être utilisé avec la canule (100) selon l'une quelconque des revendications 1 à 11, l'obturateur (300) comprenant une partie poignée, une pointe (310), un corps (320), et un arbre (330) reliant la pointe (310) au corps (320), le corps (320) de l'obturateur (300) comprenant un moyen de commande (400) qui comprend au moins une partie inclinée (401, 402) prévue dans la partie poignée (320) de l'obturateur (300), la partie inclinée (401, 402) étant configurée pour entraîner la partie d'entrée de la soupape de régulation de fluide (233) de la canule latéralement par rapport à l'axe de l'alésage (103) de la canule (100), afin d'actionner la soupape de régulation de fluide (233).

13. Procédé de fabrication d'un trocart de laparoscopie selon l'une quelconque des revendications 1 à 11 ou obturateur selon la revendication 12, comprenant les étapes suivantes :
fourniture d'un support de stockage lisible par ordinateur contenant des données représentant un modèle tridimensionnel approprié pour une utilisation dans la fabrication du trocart de laparoscopie ou de l'obturateur, respectivement ; et
fabrication du trocart de laparoscopie ou de l'obturateur, respectivement, à l'aide d'instructions contenues dans le modèle tridimensionnel.

14. Support lisible par ordinateur contenant des données représentant un modèle tridimensionnel du trocart de laparoscopie selon l'une quelconque des revendications 1 à 11 pour le procédé de fabrication d'un trocart de laparoscopie selon la revendication 13, ou de l'obturateur (300) selon la revendication 12 pour le procédé de fabrication d'un obturateur selon la revendication 13.
